# EUROPEAN PATENT APPLICATION

(11) **EP 4 620 507 A1**
(43) Date of publication of application: **24.09.2025**
(21) Application number: 23900063.1
(22) Date of filing: 07.12.2023
(51) Int. Cl.: A61M 37/00, A61B 18/00, A61B 18/04, A61B 18/12, A61B 18/14, A61N 1/32, A61N 1/18

(54) **CONTROL METHOD FOR MICRONEEDLE THERAPEUTIC HEAD AND MICRONEEDLE THERAPEUTIC INSTRUMENT**

(30) Priority: 09.12.2022 CN 202211593311
(71) Applicant: Shenzhen Peninsula Medical Group, Shenzhen, Guangdong 518000 (CN)
(72) Inventor: LIANG, Yongsheng, Shenzhen, Guangdong 518000 (CN); LI, Yanan, Shenzhen, Guangdong 518000 (CN); DING, Yi, Shenzhen, Guangdong 518000 (CN); LEI, Xiaobing, Shenzhen, Guangdong 518000 (CN)
(74) Representative: Osterhoff, Utz
(86) International application number: PCT/CN2023/137157
(87) International publication number: WO 2024/120494

(57) **Abstract**

A control method for a microneedle treatment tip and a microneedle treatment device. The control method for the microneedle treatment tip is applied to the microneedle treatment device, and the microneedle treatment device includes the microneedle treatment tip and a coolant delivery control mechanism. The microneedle treatment tip is provided with a microneedle (20) and a treatment surface, and the coolant delivery control mechanism is configured to deliver coolant to the treatment surface. The control method for the microneedle treatment tip includes: when the microneedle (20) is in a working state, delivering, via the coolant delivery control mechanism, the coolant to the treatment surface in a first preset mode.

## Description

### CROSS-REFERENCE TO RELATED APPLICATIONS

The present application claims priority to Chinese Patent Application No. 202211593311.4, filed on December 9, 2022, the entire contents of which are incorporated herein by reference.

### TECHNICAL FIELD

The present application relates to treating dermatological tissue, and in particular to a control method for a treatment tip and a treatment device.

### BACKGROUND

The treatment tip is a medical device that uses the needle to generate electrical current in the subcutaneous fat layer, thereby destroying fat cells and achieving fat reduction. It needs to generate the electrical current, this results in an increase in skin temperature. In order to avoid scalding the superficial skin during treatment or to reduce skin pain, it is usually necessary to cool the superficial skin. In the prior art, a cold-conducting component is usually provided at the contact point between the treatment tip and the skin, and the temperature of the cold-conducting component is reduced through semiconductor refrigeration, thereby lowering the temperature of the superficial skin. Therefore, the existing control method of the treatment tip can only control the cooling power of the semiconductor, so that the skin can have a sufficient cooling effect during the treatment process. However, it is limited by the power of the semiconductor on the one hand and limited by the cold-conducting speed of the cold-conducting component on the other hand, resulting in low cooling efficiency, and making it difficult to meet the need for cooling skin during treatment.

### SUMMARY

The main purpose of the present application is to provide a control method for a treatment tip, aiming to improve the cooling effect of the treatment tip.

In order to achieve the above purpose, the control method for the treatment tip is applied to a treatment device. The treatment device includes a treatment tip and a coolant delivery control mechanism; the needle and the treatment surface are provided on the treatment tip, and the coolant delivery control mechanism is configured to deliver coolant to the treatment surface.

The control method for the treatment tip includes: during that a needle is in a working state, delivering, via the coolant delivery control mechanism, the coolant to the treatment surface in a first preset mode.

In an embodiment, the first preset mode is that the coolant delivery control mechanism continuously delivers the coolant to the treatment surface during a first preset time and the coolant delivery control mechanism stops delivering the coolant during a second preset time, and reacting circular progress; or
the treatment device also includes a temperature detector, and the temperature detector is provided on the treatment tip and is configured to obtain a temperature of skin; during that the temperature of the skin exceeds a first preset temperature, the coolant delivery control mechanism delivers the coolant to the treatment surface; during that the temperature of the skin is lower than the first preset temperature, the coolant delivery control mechanism stops delivering the coolant to the treatment surface; or
the coolant delivery control mechanism injects the coolant to the treatment surface a preset number of times.

In an embodiment, before the needle starts to work, delivering, via the coolant delivery control mechanism, the coolant to the treatment surface in a second preset mode.

In an embodiment, the second preset mode is that the coolant delivery control mechanism delivers the coolant to the treatment surface during a third preset time; or
the treatment device also includes a temperature detector; the temperature detector is provided on the treatment tip and is configured to obtain a temperature of skin, and the coolant delivery control mechanism continuously delivers the coolant to the treatment surface until the temperature of the skin is less than or equal to a second preset temperature.

In an embodiment, after the needle stops working, delivering, via the coolant delivery control mechanism, the coolant to the treatment surface in a third preset mode.

In an embodiment, the third preset mode is that the coolant delivery control mechanism delivers the coolant to the treatment surface during a fourth preset time; or
the treatment device further includes a temperature detector; the temperature detector is provided on the treatment tip and is configured to obtain a temperature of skin, and the coolant delivery control mechanism continuously delivers the coolant to the treatment surface until the temperature of the skin is less than or equal to a third preset temperature.

In an embodiment, the treatment device further includes a negative pressure control mechanism; the treatment tip is also provided with a negative pressure groove, and the negative pressure groove is provided on the treatment surface; an opening of the negative pressure groove is configured to face the treatment surface, and the negative pressure control mechanism is configured to provide the negative pressure to the negative pressure groove. The control method for the treatment tip also includes: before the needle starts to work, starting to provide negative pressure by the negative pressure control mechanism; and/or
the treatment device further includes a temperature detector, the temperature detector is provided on the treatment tip, during that the needle stops working, the temperature detector obtains the temperature of the skin, and after the temperature of the skin is less than or equal to a fourth preset temperature, the negative pressure control mechanism stops providing the negative pressure to the negative pressure groove.

In an embodiment, a negative pressure tube is provided on the treatment tip, the negative pressure tube is connected to the negative pressure groove, and the negative pressure control mechanism is connected to the negative pressure tube. The control method for the treatment tip includes: before the needle starts to work, the negative pressure control mechanism starts to extract the gas in the negative pressure groove through the negative pressure tube to provide negative pressure to the negative pressure groove; and/or
the treatment device further includes a temperature detector, the temperature detector is provided on the treatment tip, during that the needle stops working, the temperature detector obtains the temperature of the skin, and after the temperature of the skin is less than or equal to a fourth preset temperature, the negative pressure control mechanism stops extracting the gas in the negative pressure groove through the negative pressure tube, to stop providing the negative pressure to the negative pressure groove.

In an embodiment, the treatment tip includes a housing, a coolant tube and a needle. A needle cavity is provided in the housing, the needle is installed in the needle cavity, the coolant tube is communicated with the needle cavity, the treatment surface is provided on the housing, and a needle outlet is provided on the treatment surface. The needle outlet is configured to allow the needle to move back and forth and protrude out of the treatment surface. The treatment surface is also provided with a coolant outlet. The distance between the needle outlet and the coolant outlet is configured as a needle-coolant outlet gap. The coolant outlet penetrates the housing where the treatment surface is located, and is communicated with the needle cavity. The control method of the treatment tip includes: the coolant delivery control mechanism is communicated with the coolant tube, when the needle is in the working state, the coolant delivery control mechanism delivers coolant to the coolant tube through a first preset mode, the coolant tube delivers the coolant to the needle cavity, and the coolant flows to the treatment surface through the coolant outlet or needle-coolant outlet gap.

In an embodiment, the treatment device further includes a pressure sensor, and the pressure sensor is provided on the treatment tip. The control method for the treatment tip includes:
acquiring, via the pressure sensor, a pressure between the treatment surface and skin; and
during that the pressure between the treatment surface and the skin is greater than or equal to a first preset pressure, delivering, via the coolant delivery control mechanism, the coolant to the treatment surface in a second preset mode; or
the treatment device further includes the negative pressure control mechanism; the treatment tip is also provided with the negative pressure groove, and the negative pressure groove is provided on the treatment surface; an opening of the negative pressure groove is configured to face the treatment surface, and the negative pressure control mechanism is configured to provide the negative pressure to the negative pressure groove.

The control method for the treatment tip includes:
after the negative pressure control mechanism starts to provide negative pressure to the negative pressure groove, delivering, via the coolant delivery control mechanism, the coolant to the treatment surface in a second preset mode.

In an embodiment, the treatment surface is provided with the needle outlet, the needle outlet is configured to allow the needle to move back and forth and protrude out of the treatment surface and puncture into the skin, and a needle-coolant outlet gap is provided between the needle outlet and the coolant outlet. The control method for the treatment tip includes: during that the needle is in the working state, delivering, via the coolant delivery control mechanism, the coolant to the needle-coolant outlet gap in a first preset mode, the coolant flows to the treatment surface through the needle-coolant outlet gap; or
the treatment surface is provided with the coolant outlet, and the control method for the treatment tip includes:
during that the needle is in the working state, delivering, via the coolant delivery control mechanism, the coolant to the coolant outlet in a first preset mode, the coolant flows to the treatment surface through the coolant outlet; or
the treatment surface is provided with a needle outlet slot, and the needle outlet slot is configured as a slot having a particular size facing the treatment surface, and the control method for the treatment tip includes:
   during that the needle is in the working state, delivering, via the coolant delivery control mechanism, the coolant to the needle outlet slot, the coolant flows to the treatment surface through the needle outlet slot.

The present application also provides a treatment device, the treatment device includes the treatment tip, and the treatment tip is controlled through the control method for the treatment tip.

The technical solution of the present application uses the coolant delivery control mechanism to deliver coolant to the treatment surface through the first preset mode to improve the cooling effect of the treatment tip. Since the coolant itself has a low temperature and can cool the skin, and after the coolant is delivered to the treatment surface, it can directly act on the skin. Therefore, the coolant is not limited by the cooling speed of the cooling device, nor is it limited by the solid heat conduction speed. Under the control of the first preset mode, a sufficient amount of coolant can be directly delivered to the treatment surface to quickly reduce the skin temperature and achieve rapid cooling. Therefore, the technical solution of the present application can improve the cooling effect of the treatment tip.

### BRIEF DESCRIPTION OF THE DRAWINGS

In order to more clearly explain the embodiments of the present application or the technical solutions in the related art, the drawings needed to be used in the description of the embodiments or the related art will be briefly introduced below. Obviously, the accompanying drawings in the following description are only some embodiments of the present application. For those skilled in the art, other drawings can be obtained based on the structures shown in these drawings without creative efforts.
FIG. 1 is a schematic structural diagram of a treatment tip in a treatment device according to an embodiment of the present application.
FIG. 2 is a partial enlarged view at point A in FIG. 1.
FIG. 3 is a schematic structural diagram of the embodiment in FIG. 1 from another perspective.
FIG. 4 is a schematic cross-sectional structural diagram along the B-B direction in FIG. 3.
FIG. 5 is a schematic structural diagram of the treatment tip in the treatment device according to an embodiment of the present application.
FIG. 6 is a schematic cross-sectional structural diagram along the C-C direction in FIG. 5.
FIG. 7 is a schematic structural diagram of the treatment tip in the treatment device according to an embodiment of the present application.
FIG. 8 is a schematic structural diagram of the treatment tip in the treatment device according to an embodiment of the present application.
FIG. 9 is a schematic structural diagram of the treatment tip in the treatment device according to an embodiment of the present application.

Description of reference signs:

| reference sign | name | reference sign | name |
|---|---|---|---|
| 10 | housing | 15 | coolant outlet |
| 11 | needle cavity | 16 | needle column |
| 12 | needle outlet | 20 | needle |
| 13 | guide rib | 30 | coolant tube |
| 14 | negative pressure groove | 40 | negative pressure tube |

The realization of the purpose, functional features and advantages of the present application will be further described in conjunction with the embodiments, with reference to the accompanying drawings.

### DETAILED DESCRIPTION OF THE EMBODIMENTS

The technical solutions in the embodiments of the present application will be clearly and completely described below with reference to the accompanying drawings in the embodiments of the present application. Obviously, the described embodiments are only some rather than all of the embodiments of the present application. Based on the embodiments in the present application, all other embodiments obtained by those skilled in the art without creative efforts fall within the scope of the present application.

It should be noted that all directional indications (such as up, down, left, right, front, back...) in the embodiments of the present application are only used to explain the relative positional relationship between components in a specific posture (as shown in the drawings), movement conditions, etc. If the specific posture changes, the directional indication will also change accordingly.

In addition, descriptions involving "first", "second", etc. in the present application are for descriptive purposes only and cannot be understood as indicating or implying their relative importance or implicitly indicating the number of indicated technical features. Therefore, features defined as "first" and "second" can explicitly or implicitly include at least one of these features. In addition, the technical solutions in the various embodiments can be combined with each other, but it must be based on what those skilled in the art can implement. When the combination of technical solutions is contradictory or cannot be realized, it should be considered that such a combination of technical solutions does not exist, nor is it within the scope of the present application.

The present application provides a control method for a treatment tip.

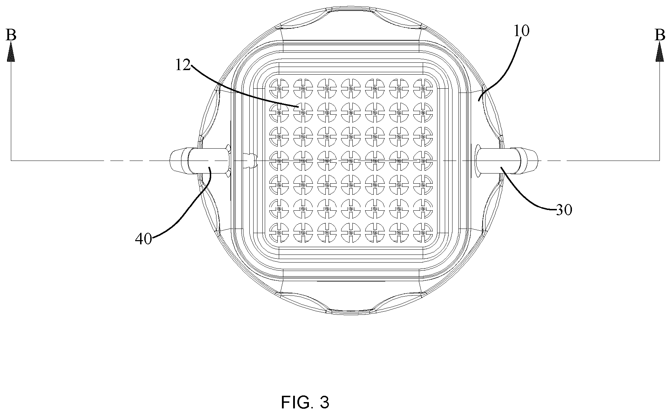

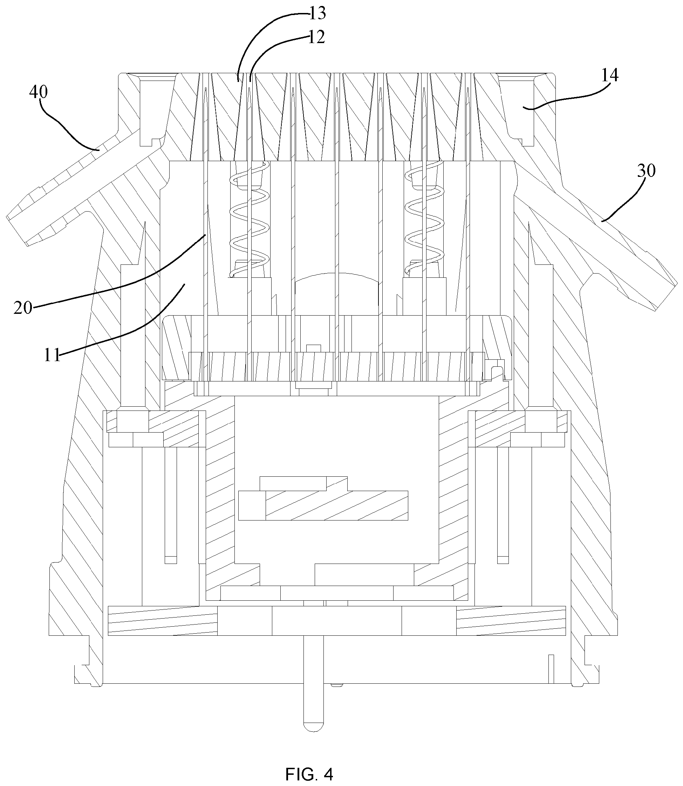

In the embodiment of the present application, as shown in FIG. 3 and FIG. 4, the control method for the treatment tip is applied to a treatment device. The treatment device includes a treatment tip and a coolant delivery control mechanism. The treatment tip is provided with a needle 20 and a treatment surface. The coolant delivery control mechanism is configured to deliver coolant to the treatment surface. The control method for the treatment device includes: when the needle 20 is in a working state, the coolant delivery control mechanism delivers coolant to the treatment surface in a first preset mode.

The coolant delivery control mechanism can have a coolant tank to store the coolant and an air pump to pump out the coolant from the coolant tank, and adjust the coolant delivery volume by adjusting the power of the air pump; it can also have a valve with an adjustable opening. The atomized coolant is pushed out through the pressure generated by the natural atomization of the coolant, and the coolant delivery volume is changed by adjusting the opening degree of the valve. The control for the power of the atomizing pump and the control for the opening degree of the valve belong to the existing technology, and the structure and principle will not be described again. The coolant can be a gas from at least one of hydrogen, nitrogen and helium and so on, which is stored in liquid form in the storage tank. The first preset mode can be that the coolant is continuously delivered to the treatment surface when the needle 20 is in a working state until the needle 20 stops working. The treatment surface is a surface in contact with the skin. When the needle 20 works, the needle 20 first penetrates the skin and reach the subcutaneous fat layer, and then start to release radio frequency current, thereby destroying subcutaneous fat cells and reducing fat. When the coolant is delivered to the treatment surface, it can directly act on the skin. When the needle 20 is in the working state, it means that the needle 20 has penetrated into the skin and has not been withdrawn.

Referring to FIG. 3 and FIG. 4, in an embodiment, the first preset mode is that the coolant delivery control mechanism continuously delivers the coolant to the treatment surface during the first preset time, then stops delivering the coolant to the treatment surface during the second preset time and reacts the circular progress.

Alternatively, the treatment device also includes a temperature detector. The temperature detector is provided on the treatment tip. The temperature detector obtains the temperature of the skin. When the temperature of the skin exceeds a first preset temperature, the coolant delivery control mechanism delivers the coolant to the treatment surface, and when the temperature of the skin is lower than the first preset temperature, the coolant delivery control mechanism stops delivering the coolant to the treatment surface; or
the coolant delivery control mechanism sprays the coolant to the treatment surface a preset number of times.

The first preset time and the second preset time form a cycle. During the working time of the needle 20, the coolant is continuously supplied in this cycle to prevent the skin temperature from being too low or too high, thereby protecting the skin from damage and reducing pain.

The temperature detector can be an infrared temperature sensor, which can be provided in a needle cavity 11, so that the temperature of the skin can be measured through a coolant through the outlet. The temperature detector can also be a thermosensitive resistor. The thermosensitive resistor can be provided at the tip of a certain needle 20, and the needle 20 provided with the thermosensitive resistor is configured to be shorter than the other needles 20. When the needle 20 acts, the needle 20 does not penetrate the skin, but only contacts the skin surface, so that data of the skin temperature can be obtained by measuring the resistance of the thermosensitive resistor. When the skin temperature exceeds the first preset temperature, the coolant is delivered to the skin. When the skin temperature is lower than the first preset temperature, the coolant delivery is stopped. This helps save coolant and improves intelligence of the treatment device. The first preset temperature can be a temperature range. When the temperature goes from low to high and exceeds the upper limit of the range, the coolant injection starts. When the temperature goes from high to low and exceeds the lower limit of the range, the coolant injection stops. This helps avoid frequent injection of coolant, thus protecting the coolant delivery control mechanism.

When the needle is in the working state, the coolant delivery control mechanism sprays the coolant to the treatment surface a preset number of times. The preset number of times can be set manually or fixed when the treatment device is produced, which is not limited here. There is no limit to the injection pattern here, that is, as the needle works, the injection frequency can be gradually increased, and finally when the preset number of injections is reached, the needle completes the work at the same time; it can also be when the needle starts to work, the injection frequency is high. As the needle works, the injection frequency is gradually reduced. Finally, when the preset number of injections is reached, the needle completes the work at the same time; it can also be irregular and random injection.

Referring to FIG. 3 and FIG. 4, in an embodiment, before the needle 20 starts to work, the coolant delivery control mechanism delivers the coolant to the treatment surface through the second preset mode. Before the needle 20 starts to work, the skin is at room temperature. At this temperature, pain receptors on the skin can work normally, making the skin more sensitive to pain. At this time, the coolant is delivered to lower the skin temperature and reduce the activity of pain receptors on the skin, thereby reducing pain and improving the user's experience. Before the needle 20 starts to work refers to before the needle 20 starts to release radio frequency current to destroy subcutaneous fat cells. At this time, the needle 20 may not have penetrated the skin, or may have penetrated the skin.

Referring to FIG. 3 and FIG. 4, in an embodiment, the second preset mode is: the coolant delivery control mechanism delivers the coolant to the treatment surface during a third preset time; or
the treatment device also includes a temperature detector. The temperature detector is provided on the treatment tip. The temperature detector obtains the temperature of the skin. The coolant delivery control mechanism continuously delivers the coolant to the treatment surface until the temperature of the skin is less than or equal to the second preset temperature.

The second preset mode can be that the coolant is continuously delivered during the third preset time to ensure that the skin temperature is lowered, thereby reducing pain. The second preset mode can also be when the coolant is delivered until the temperature detector detects that the skin temperature is lower than the second preset temperature. The setting method of the temperature detector refers to the above embodiment.

Referring to FIG. 3 and FIG. 4, in an embodiment, after the needle 20 stops working, the coolant delivery control mechanism delivers the coolant to the treatment surface through the third preset mode.

After the needle 20 stops working, the temperature of the subcutaneous fat layer is still high, and the heat of the subcutaneous fat layer may gradually be conducted to the dermis layer, thereby increasing the activity of pain receptors in the skin, and causing pain to the user. When the needle 20 stops working, the coolant is continuously delivered, which can further ensure that the skin temperature remains low for a longer period of time. After the needle 20 stops working, it means that the needle 20 stops releasing radio frequency current and completes the destruction of subcutaneous fat cells. At this time, the needle 20 may still be in the skin, or may have been pulled out of the skin.

Referring to FIG. 3 and FIG. 4, in an embodiment, the third preset mode is: the coolant delivery control mechanism delivers the coolant to the treatment surface during the fourth preset time; or
the treatment device also includes a temperature detector, and the temperature detector is provided on the treatment tip. The temperature detector obtains the temperature of the skin. The coolant delivery control mechanism continuously delivers the coolant to the treatment surface until the temperature of the skin is less than or equal to the third preset temperature.

The third preset mode can be that the coolant is delivered during the fourth preset time to further reduce the temperature of the skin, thereby offsetting the heat transferred from the subcutaneous fat layer; it can also be that the temperature of the skin is detected through the temperature detector and the coolant is delivered until the temperature of the skin is less than or equal to the third preset temperature, which is beneficial to saving coolant.

Referring to FIG. 3 and FIG. 4, in an embodiment, the treatment device also includes a negative pressure control mechanism. The treatment tip is also provided with a negative pressure groove 14. The negative pressure groove 14 is provided on the treatment surface. The opening of the negative pressure groove 14 faces the treatment surface, and the negative pressure control mechanism is used to provide negative pressure to the negative pressure groove 14. The control method for the treatment device also includes: before the needle 20 starts to work, the negative pressure control mechanism starts to provide negative pressure; and/or
the treatment device also includes the temperature detector. The temperature detector is provided on the treatment tip. When the needle 20 stops working, the temperature detector obtains the temperature of the skin. When the temperature of the skin is less than or equal to the fourth preset temperature, the negative pressure control mechanism stops providing negative pressure to the negative pressure groove 14.

Before the needle 20 starts to work and before the needle 20 penetrates into the skin, the negative pressure is provided to the negative pressure groove 14 through the negative pressure control mechanism. Since the treatment surface faces the skin, when there is negative pressure in the negative pressure groove 14, the treatment tip is made to suck the skin tightly and fix on the skin, thereby ensuring the stabilization of the insertion direction of the needle 20. After the treatment tip stops working, the coolant is further delivered to reduce the temperature of the skin, therefore the temperature detector can be configured to detect the temperature of the skin until the temperature of the skin is lower than the fourth preset temperature. Then, the negative pressure is released, so that the user can continuously deliver the coolant to the skin. The setting method of the temperature detector refers to the above embodiment. The fourth preset temperature can be set to be equal to the third preset temperature. The negative pressure control mechanism can be a mechanism with an air pump. The gas in the negative pressure groove 14 is extracted by the air pump to provide the negative pressure to the negative pressure groove 14. The control structure and method for the air pump belong to the existing technology, and the negative pressure control mechanism can also be an air suction bag. The user presses the air suction bag to generate negative pressure and sucks the gas in the negative pressure groove 14 to provide negative pressure to the negative pressure groove 14.

Referring to FIG. 3 and FIG. 4, in an embodiment, a negative pressure tube 40 is provided on the treatment tip. The negative pressure tube 40 is connected to the negative pressure groove 14. The negative pressure control mechanism is connected to the negative pressure tube 40. The specific control method for the treatment device is as follows: before the needle 20 starts to work, the negative pressure control mechanism begins to extract the gas in the negative pressure groove 14 through the negative pressure tube 40 to provide negative pressure to the negative pressure groove 14; and/or
the treatment device also includes a temperature detector. The temperature detector is provided on the treatment tip. When the needle 20 stops working, the temperature detector obtains the temperature of the skin. When the temperature of the skin is less than or equal to the fourth preset temperature, the negative pressure control mechanism stops extracting the gas in the negative pressure groove 14 through the negative pressure tube 40 to stop providing negative pressure to the negative pressure groove 14.

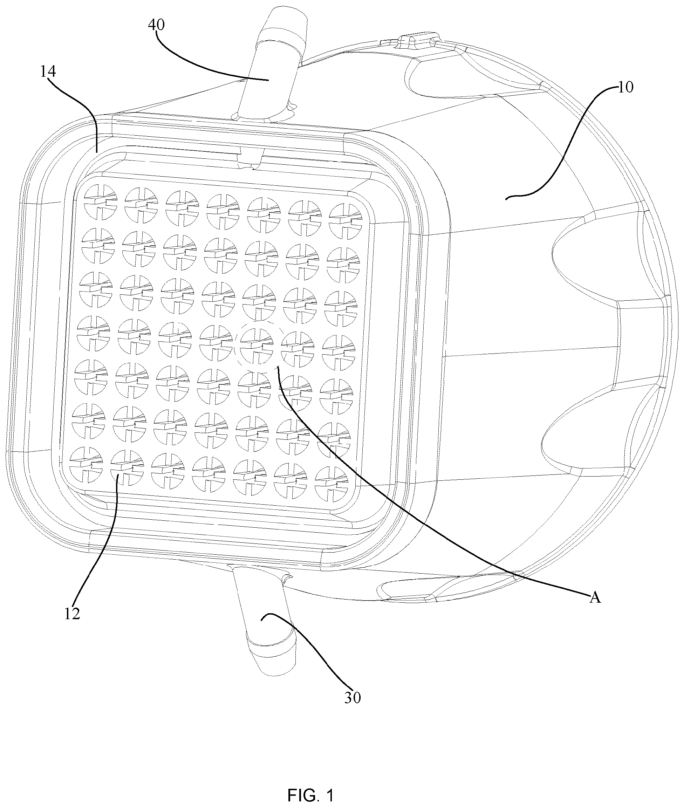

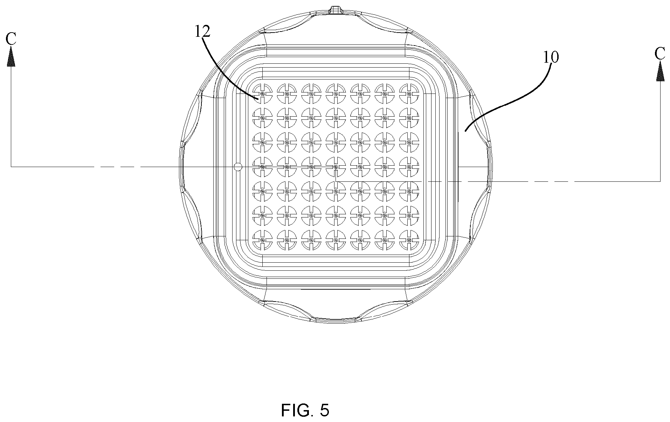

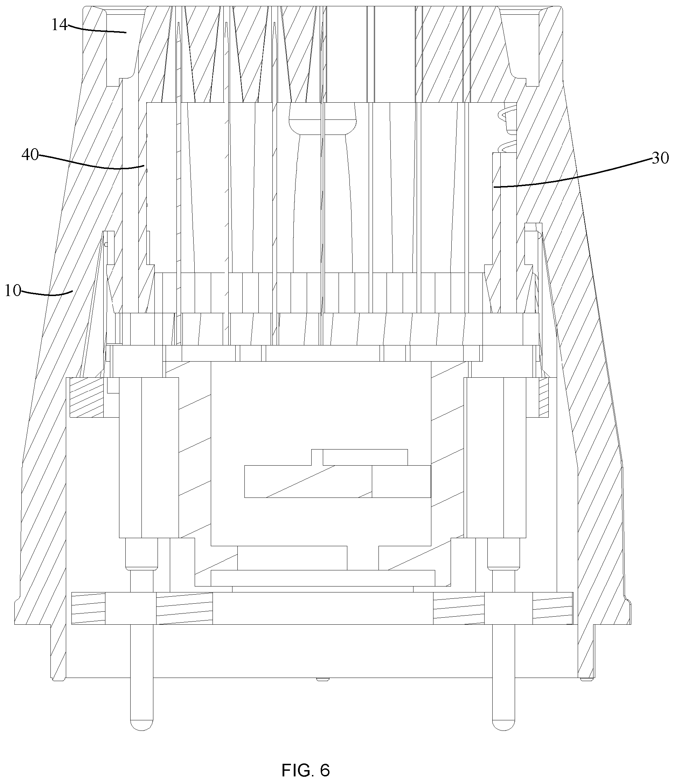

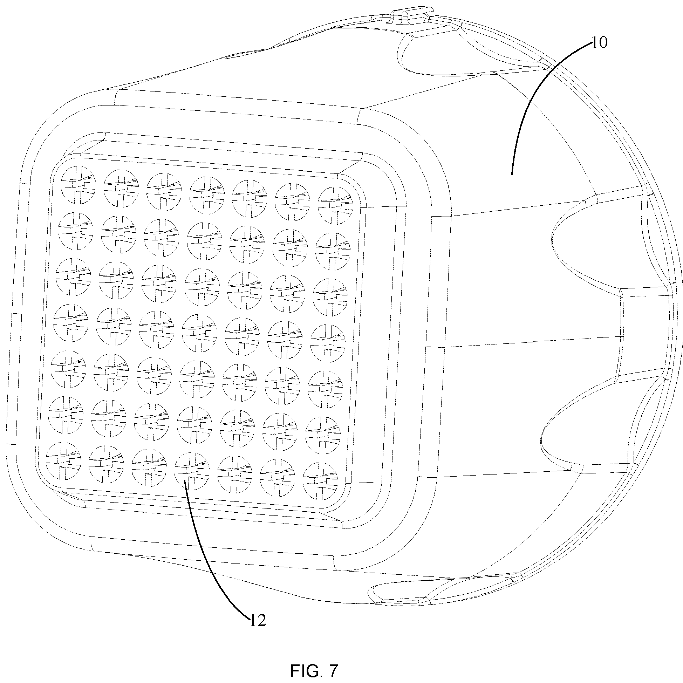

The arrangement of the negative pressure tube 40 can facilitate the communication between the negative pressure control mechanism and the negative pressure groove 14. The arrangement of the temperature detector refers to the above embodiment. Referring to FIG. 1, the negative pressure tube 40 can be provided outside a housing 10 of the treatment tip, so that a pipeline of the negative pressure control mechanism can be easily communicated with the negative pressure tube 40. Referring to FIG. 5 to FIG. 7, the negative pressure tube 40 can also be provided inside the housing 10, so that the wiring of the negative pressure tube 40 cannot block the user's view, making it convenient for the user to treat the patient.

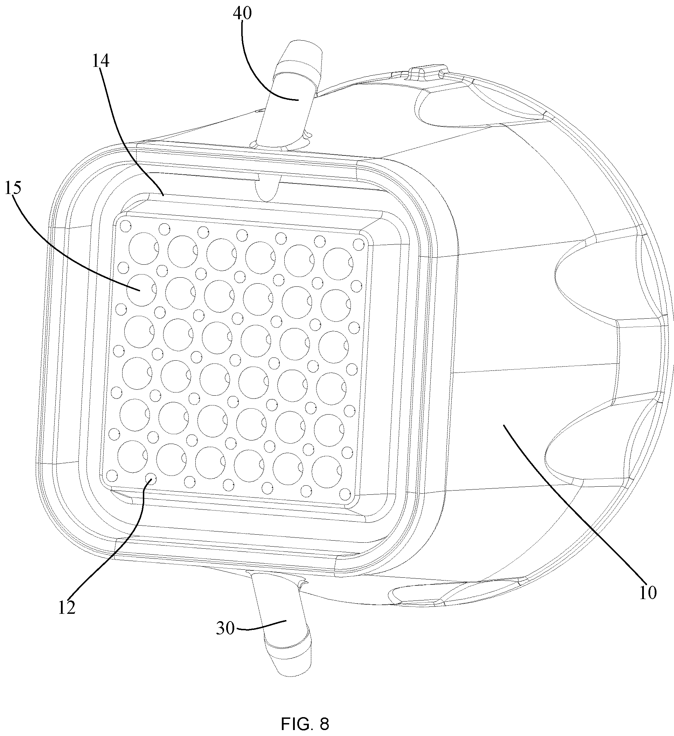

Referring to FIG. 1 to FIG. 9, in an embodiment, the treatment tip includes a housing 10 and a coolant tube 30. A needle cavity 11 is provided in the housing 10. The needle 20 is installed in the needle cavity 11. The coolant tube 30 is communicated with the needle cavity 11. The treatment surface is provided on the housing 10. The treatment surface is provided with a needle outlet 12. The needle outlet 12 is used to allow the needle 20 to move back and forth and protrude out of the treatment surface. The treatment surface is also provided with a coolant outlet 15. The distance between the needle outlet 12 and the coolant outlet 15 is configured as a needle-coolant outlet gap. The coolant outlet 15 penetrates the housing 10 where the treatment surface is located and is communicated with the needle cavity 11. The specific control method for the treatment device is as follows: the coolant delivery control mechanism is connected to the coolant tube 30, when the needle 20 is in the working state, the coolant delivery control mechanism delivers the coolant to the coolant tube 30 through the first preset mode, the coolant tube 30 delivers the coolant to the needle cavity 11, and the coolant flows to the treatment surface through the coolant outlet 15 or the needle-coolant outlet gap.

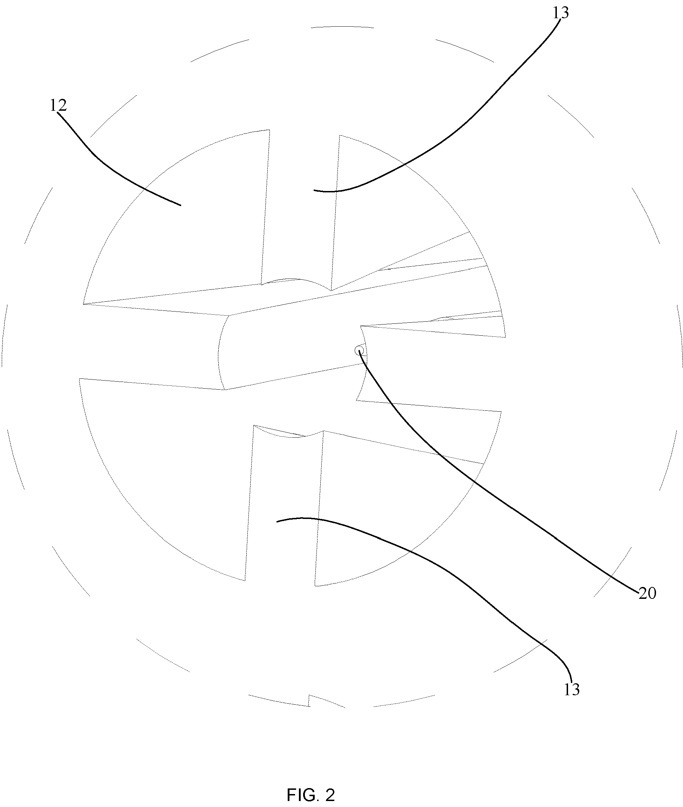

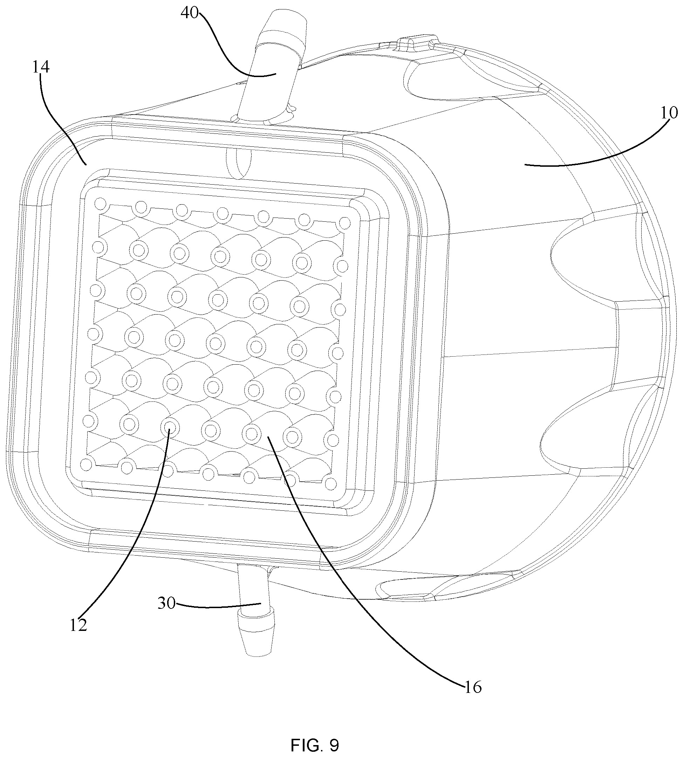

The coolant tube 30 can conveniently allow the coolant delivery control mechanism to deliver the coolant to the treatment surface. The coolant tube 30 is communicated with the needle cavity 11. The coolant delivery control mechanism is communicated with the coolant tube 30, that is, communicated with the needle cavity 11, and delivers the coolant into the needle cavity 11. Since the coolant outlet 15 penetrates the housing 10 and is communicated with the needle cavity 11, and provided on the treatment surface, the coolant can pass through the coolant outlet 15 and diffuse to the treatment surface, thereby delivering the coolant to the treatment surface. The coolant can also diffuse onto the treatment surface from the needle-coolant outlet gap. As shown in FIG. 2, since the needle 20 is usually thin and soft, they need support to prevent the needle 20 from bending when penetrating the skin. Therefore, a guide rib 13 is provided on the needle outlet 12 to support the needle 20. Referring to FIG. 9, the coolant tube 30 may not be communicated with the needle cavity 11. In the embodiment shown in FIG. 9, a needle outlet slot is provided on the treatment surface. The needle outlet slot is used to accommodate a needle column 16. The coolant tube 30 is communicated with the needle outlet slot, thereby delivering the coolant to the treatment surface and cooling the skin.

Referring to FIG. 1, in an embodiment, the treatment device further includes a pressure sensor. The pressure sensor is provided on the treatment tip. The specific control method for the treatment tip is as follows: the pressure sensor acquires a pressure between the treatment surface and the skin, when the pressure between the treatment surface and the skin is greater than or equal to a first preset pressure, the coolant delivery control mechanism delivers the coolant to the treatment surface through the second preset mode; or
the treatment device also includes a negative pressure control mechanism. The treatment tip is also provided with a negative pressure groove 14. The negative pressure groove 14 is provided on the treatment surface. An opening of the negative pressure groove 14 faces the treatment surface. The negative pressure control mechanism is used to provide negative pressure to the negative pressure groove 14. The specific control method for the needle treatment tip is as follows: after the negative pressure control mechanism starts to provide the negative pressure to the negative pressure groove 14, the coolant delivery control mechanism delivers the coolant to the treatment surface through the second preset mode.

The pressure sensor can be provided on the treatment surface. The pressure sensor can be a diaphragm pressure sensor to improve the contact effect between the treatment surface and the skin. The pressure sensor can also be a micro switch, which triggers the micro switch to indicate that the contact pressure between the skin and the treatment surface is sufficient. Since the needles need to be inserted into the skin in usage, a certain pressure is required to press the treatment surface against the skin to ensure the accuracy of the needle insertion process and avoid deviation from the desired treatment position during the needle insertion process. The pressure sensor can prompt the user that the pressure is sufficient to start treatment, making it easier for the user to operate. The coolant delivery control mechanism can save coolant by supplying the coolant after the pressure is sufficient, because when the pressure is insufficient, the user will not start treatment and may only be in the preparation stage. At this time, the coolant is released too early, which will lead to waste.

The negative pressure groove 14 can make the treatment surface close to the skin. At the same time, the user will start treatment only after the treatment surface is close to the skin. Therefore, the negative pressure delivery control mechanism provides the coolant after there is negative pressure in the negative pressure groove 14, which can save the coolant and avoid premature release of the coolant.

Referring to FIG. 1, FIG. 8 and FIG. 9, in an embodiment, the needle outlet 12 is provided on the treatment surface. The needle outlet 12 is used to allow the needle 20 to protrude out of the treatment surface and penetrate the skin. The needle-coolant outlet gap is provided between the needle outlet 12 and the coolant outlet 15. The specific control method for the treatment tip is as follows: when the needle 20 is in the working state, the coolant delivery control mechanism delivers the coolant to the needle-coolant outlet gap through the first preset mode, and the coolant flows to the treatment surface through the needle-coolant outlet gap; or
the coolant outlet 15 is provided on the treatment surface. The specific control method for the treatment tip is as follows: when the needle 20 is in the working state, the coolant delivery control mechanism delivers the coolant to the coolant outlet 15 through the first preset mode, and the coolant flows to the treatment surface through the coolant outlet 15; or
the needle outlet slot is provided on the treatment surface, and the needle outlet slot is configured as a slot having a particular size facing the treatment surface. The specific control method for the treatment tip is as follows: when the needle 20 is in the working state, the coolant delivery control mechanism delivers the coolant to the needle outlet slot, and the coolant flows to the treatment surface through the needle outlet slot.

As shown in FIG. 9, the opening of the open needle slot is exactly formed on the treatment surface, which expands the contact area between the coolant and the skin, and can improve the cooling effect. The coolant tube 30 can be communicated with the needle slot, so that the coolant delivery control mechanism can output the coolant to the needle slot.

When the coolant is outputted to the coolant outlet 15, a separate coolant cavity can also be provided for the coolant to control the flow direction of the coolant.

For example, a cover plate is used to close the opening of the needle slot, and a number of coolant outlets 15 are provided on the cover plate. In this way, the cover plate, the side walls and the bottom wall of the needle outlet slot can form a coolant cavity. At this time, the coolant tube is communicated with the coolant cavity, and the coolant is discharged from the coolant outlet 15 and flows toward the treatment surface.

The present application also provides a treatment device. The treatment device includes a treatment tip, and the treatment tip is controlled by the above-mentioned control method for the treatment tip. For the specific content of the control method for the treatment tip, please refer to the above-mentioned embodiments. Since this treatment device adopts all the technical solutions of the above embodiments, it has at least all the beneficial effects brought by the technical solutions of the above embodiments, which will not be described again here.

The above are only some embodiments of the present application, and are not intended to limit the scope of the present application. Under the inventive concept of the present application, any equivalent structural transformations made using the contents of the description and the accompanying drawings of the present application, or direct or indirect applications in other related technical fields, are all included in the scope of the present application.

## Claims

1. A control method for a treatment tip, applied to a treatment device, **characterized by** comprising:
during that a needle is in a working state, delivering, via a coolant delivery control mechanism, coolant to a treatment surface in a first preset mode;
wherein the treatment device comprises a treatment tip and the coolant delivery control mechanism; the needle and the treatment surface are provided on the treatment tip, and the coolant delivery control mechanism is configured to deliver the coolant to the treatment surface.

2. The control method for the treatment tip according to claim 1, wherein the first preset mode is that the coolant delivery control mechanism continuously delivers the coolant to the treatment surface during a first preset time and the coolant delivery control mechanism stops delivering the coolant during a second preset time, and reacting circular progress; or
the treatment device also comprises a temperature detector, and the temperature detector is provided on the treatment tip and is configured to obtain a temperature of skin; during that the temperature of the skin exceeds a first preset temperature, the coolant delivery control mechanism delivers the coolant to the treatment surface; during that the temperature of the skin is lower than the first preset temperature, the coolant delivery control mechanism stops delivering the coolant to the treatment surface; or
the coolant delivery control mechanism injects the coolant to the treatment surface a preset number of times.

3. The control method for the treatment tip according to claim 1, wherein before the needle starts to work, the coolant delivery control mechanism delivers the coolant to the treatment surface in a second preset mode.

4. The control method for the treatment tip according to claim 3, wherein the second preset mode is that the coolant delivery control mechanism delivers the coolant to the treatment surface during a third preset time; or
the treatment device also comprises a temperature detector; the temperature detector is provided on the treatment tip and is configured to obtain a temperature of skin, and the coolant delivery control mechanism continuously delivers the coolant to the treatment surface until the temperature of the skin is less than or equal to a second preset temperature.

5. The control method for the treatment tip according to claim 3, further comprising:
acquiring, via a pressure sensor, a pressure between the treatment surface and skin; and
when the pressure between the treatment surface and the skin is greater than or equal to a first preset pressure, delivering, via the coolant delivery control mechanism, the coolant to the treatment surface in the second preset mode;
wherein the treatment device further comprises the pressure sensor, and the pressure sensor is provided on the treatment tip; or
the control method for the treatment tip further comprises:
after a negative pressure control mechanism starts to provide negative pressure to a negative pressure groove, delivering, via the coolant delivery control mechanism, the coolant to the treatment surface in the second preset mode;
wherein the treatment device further comprises the negative pressure control mechanism; the treatment tip is also provided with the negative pressure groove, and the negative pressure groove is provided on the treatment surface; an opening of the negative pressure groove is configured to face the treatment surface, and the negative pressure control mechanism is configured to provide the negative pressure to the negative pressure groove.

6. The control method for the treatment tip according to claim 1, wherein after the needle stops working, the coolant delivery control mechanism delivers the coolant to the treatment surface in a third preset mode.

7. The control method for the treatment tip according to claim 6, wherein the third preset mode is that the coolant delivery control mechanism delivers the coolant to the treatment surface during a fourth preset time; or
the treatment device further comprises a temperature detector; the temperature detector is provided on the treatment tip and is configured to obtain a temperature of skin, and the coolant delivery control mechanism continuously delivers the coolant to the treatment surface until the temperature of the skin is less than or equal to a third preset temperature.

8. The control method for the treatment tip according to claim 1, further comprising:
before the needle starts to work, starting to provide negative pressure by a negative pressure control mechanism;
wherein the treatment device further comprises the negative pressure control mechanism, the treatment tip is further provided with a negative pressure groove, the negative pressure groove is provided on the treatment surface, an opening of the negative pressure groove is configured to face the treatment surface, and the negative pressure control mechanism is configured to provide the negative pressure to the negative pressure groove; and/or
the treatment device also comprises a temperature detector, the temperature detector is provided on the treatment tip, during that the needle stops working, the temperature detector obtains the temperature of the skin, and after the temperature of the skin is less than or equal to a fourth preset temperature, the negative pressure control mechanism stops providing the negative pressure to the negative pressure groove.

9. The control method for the treatment tip according to claim 1, further comprising:
during that the needle is in the working state, delivering, via the coolant delivery control mechanism, the coolant to a needle-coolant outlet gap in a first preset mode, wherein the coolant flows to the treatment surface through the needle-coolant outlet gap;
wherein the treatment surface is provided with the needle outlet, the needle outlet is configured to allow the needle to move back and forth and protrude out of the treatment surface to puncture into the skin, and a needle-coolant outlet gap is provided between the needle outlet and the needle; or
the control method for the treatment tip further comprises:
during that the needle is in the working state, delivering, via the coolant delivery control mechanism, the coolant to a coolant outlet in a first preset mode;
wherein the coolant flows to the treatment surface through the coolant outlet; and
the treatment surface is provided with the coolant outlet; or
the control method for the treatment tip further comprises:
during that the needle is in the working state, delivering, via the coolant delivery control mechanism, the coolant to a needle outlet slot, wherein the coolant flows to the treatment surface through the needle outlet slot;
wherein the treatment surface is provided with the needle outlet slot, and the needle outlet slot is configured as a slot having a particular size facing the treatment surface.

10. A treatment device, **characterized by** comprising a treatment tip, wherein the treatment tip is controlled through the control method for the treatment tip according to any one of claims 1 to 9.
